# EUROPEAN PATENT APPLICATION

(11) **EP 4 741 805 A1**
(43) Date of publication of application: **13.05.2026**
(21) Application number: 25000107.0
(22) Date of filing: 30.10.2025
(51) Int. Cl.: G01N 21/64, G01N 21/25, G01N 21/77, G01N 21/94, G01N 21/78, G01N 33/12, G01N 1/02, G01N 1/38, G01N 33/543, G01N 1/00, C12Q 1/02, G01N 21/552

(54) **A SYSTEM FOR RAPIDLY DETECTING AND IDENTIFYING MICROORGANISMS ON BIOLOGICAL SURFACES (E.G. FISH SKIN SURFACES) AND/OR NON-BIOLOGICAL SURFACES (E.G. HABITAT OR IC SURFACES) WITH A FLEXIBLE DISSOLVABLE CLOTH AND CORRESPONDING METHODS**

(30) Priority: 12.11.2024 NL 1044990
(71) Applicant: CV Trojka tegen Pandemieën, 2215 WV Voorhout (NL)
(72) Inventor: Aalders, Maurice C. G., Amsterdam (NL); van Dam, Annemieke, Woerdense Verlaat (NL)

(57) **Abstract**

The current invention comprises systems and methods for the detection of food related bacteria, such as listeria, e. coli and s. aureus in a mobile and modular design. The combination of a dissolvable flexible cloth as sampling means, a multiple target sensor, smart readout optics and spectral processing enables to instantly ascertain, verify and validate the presence of specific microbes. The main application is the monitoring of the food and food production in order to prevent the coming to the market of bacteria invested products, such as fish, chicken or other food products destined for human consumption.

## Description

### Field (of the invention):

This invention relates to systems and corresponding methods, for rapidly detecting and identifying micro-organisms on biological surfaces (e.g. fish skin surfaces) and/or non-biological surfaces (e.g. habitat or IC surfaces), comprising specific sample means.

The invention especially finds its application in the food industry and food production industry in order to prevent the coming to the market of bacteria invested products.

The current invention comprises the technological development regarding the instant detection of food related bacteria.

### Background (of the invention):

Current: products for human consumption are normally tested in food production facilities through swabs and the results are being tested in laboratory facilities, which are located at a different location. The results are mostly only known after days. By that time the products for human consumption could already be consumed.

### Publications cited:

∘ van Dam, K. Falkena, S.A. den Daas, I. Veldhuizen. M.C.G Aalders, Improving the visualization of fingermarks using multi-target immunolabeling, For Sci Int, 324, 2021
   § Here a contrast agent was designed, based on immunolabeling, to bind to specific components in fingermarks. The contrast of the fingerprint was greatly enhanced, enabling better visualisation compared to fingerprints enhanced with conventional methods. Comparable to the current project, we target specific components with immunolabeling.
∘ L.S. Wilk, R.J.M. Hoveling, G.J. Edelman, H.J.J. Hardy, S. van Schouwen, H. van Venrooij, M.C.G. Aalders, Reconstructing the time since death using noninvasive thermometry and numerical analysis, Science Advances, 6(22), 2020.
   § An andvanced model was designed which models the energy exchange of the human body with the environment. The relevancy here is the system integration and simulation of complex processes with optimizers
∘ M. Almasian, L.S. Wilk, P.R. Bloemen, T.G. van Leeuwen, M. ter Laan, M.C.G. Aalders, Pilot feasibility study of in vivo intraoperative quantitative optical coherence tomography of human brain tissue during glioma resection, Journal of biophotonics 12 (10), e201900037, 2019.
   § By analysing the morphological buildup of gliomata at the cellular level, we were able to create contrasts between healthy and diseased tissues which are hard to distinguish otherwise.
∘ C.S. Stravers, E.L. Gool, T.G. van Leeuwen, M.C.G. Aalders, A. van Dam, Multiplex body fluid identification using surface plasmon resonance imaging with principal component analysis, Sensors and Actuators B: Chemical, 283, 355-362, 2019.
   § Here a new Plasmon resonance imaging was explored to obtain the composition of several body fluids. The technique is, again based on multiple component labelling
∘ N. Achetib, L.S. Wilk, J.C.V. Schwarz, S.A.G. Lambrechts, T.G. van Leeuwen, M.C.G. Aalders, A. van Dam, Estimating the Time of Deposition of Semen Traces using Fluorescence Protein-Lipid Oxidation Signatures, Analytical chemistry, 91(5), 3204-3208, 2019.
∘ K. Falkena, R.J.M. Hoveling, A. van Weert, S.A.G. Lambrechts, T.G. van Leeuwen, M.C.G. Aalders, A. van Dam, Prediction of DNA concentration in fingermarks using autofluorescence properties, Forensic Science International, 295, 128-136, 2019.
∘ C.S. Stravers, E.L. Gool, T.G. van Leeuwen, M.C.G. Aalders, A. van Dam, Multiplex Body Fluid Identification using Surface Plasmon Resonance Imaging with Principal Component Analysis, Sensors and Actuators B: Chemical, 2018.

### Objects and solutions of the invention

The object of the invention is to overcome at least some of the above problems.

This object is achieved with the system according to claim 1.

The object is also achieved with the system according to claim 14 to be used as a set or part of a set for sampling.

Furthermore the object is achieved with the methods according to claim 15.

Beneficial optional embodiments are the subject of the dependent claims.

With the sensitive read-out device in combination with the presumptive detection device and optional presumptive read-out device according to the invention, an on the spot detection and identification of micro-organisms can be achieved.

The systems according to the invention can be easily integrated in production facilities and used at test locations.

With the systems and methods according to the invention, rapidly detecting and identifying of micro-organisms can be materialised, especially during the production in progress in the food production facilities.

The sample means according to the invention is a dissolvable flexible cloth. The cloth can be swiped over all kinds of surfaces - even and uneven - whereby micro-organisms can easily adhere to the cloth during a swiping. The cloth could also be applied to a surface in another appropriate way, e.g. by using an appliance such as a stub. The cloth can have any appropriate shape or size. Typically the cloth is a relatively small cloth having e.g. a rectangular or square shape and e.g. a size of around 25 cm x 25cm.

With the dissolvable flexible cloth, (groups of) micro-organisms adhered thereto can be easily freed from the sample means when the flexible cloth together with the micro-organisms are dissolved in a solvent in a container. This can easily be done in the processing or sampling environment. Separation of the micro-organisms from the sample means does not have to be done any more by known techniques as washing in a remote laboratory, thus saving much time.

Further analysing can also be done in the processing or sampling environment. This obviates analysing in a remote laboratory as is done in state of the art methods, which saves enormous time.

Especially also for this reason the sensitive read out device can have a size much smaller than the stationary state of the art devices in laboratories. With the relatively small size, e.g. a volume smaller than 1 m3 e.g. 0,5 m3, 0,1 m3, or 0,01 m3, the sensitive read out device is mobile and can be easily used in process and sampling environments.

Especially with the use of the flexible cloth to which multiple different micro-organisms can adhere and with the use of multiple area sensors in the sensitive read out device, rapidly detecting and identifying of multiple different micro-organisms is made possible.

Furthermore, the dissolvement of the sample means proves the durability of the invention, since no rest product of the sample means remains.

Our invention tests the products for human consumption in-process and the results are known within minutes. Using: in-process sampling during the processing of food for human consumption.

This is preferably combined with:
- Dissolving a nitrocellulose cloth (in case of liquid sample)
- Fluorescent labeling in a container
- Confirmatory test-high sensitivity and high selectivity
- Analysis and identification using machine vision algorithms
- High specific verification through additional tests
- Instant action possibilities

These preferably fully computerized methods, using product-designed algorithms, make the detection of harmful bacteria possible within minutes. With this method the food processing facility can undertake action immediately so as to prevent that the contaminated food will be sent out further into the food distribution process.

Further benefits and advantages of the present invention will become apparent after reading the detailed description of embodiments below with appropriate reference to the accompanying figures.
Figure 1: The three step system described below.
Figure 2a: Diagram
Figure 2b and 2c: colour changes of products
Figure 3: labelling of the sample means
Figure 4: several common fluorophores with their excitation (dashed line) and emission spectra (solid line)
Figure 5: Examples of steps 1, 2 and 3 of the method

### Description of preferred embodiments of the invention

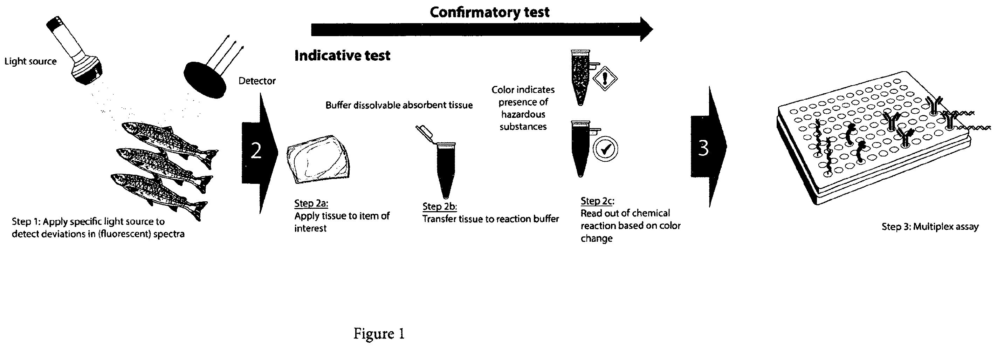

The proposed system:
Reference is made to Figure 1.

Hereafter the steps are described for a process for detecting Listeria and other types of bacteria on fish and meat surfaces in a processing environment with more explanation of the techniques involved, such as dissolution, fluorescent labeling, and advanced detection methods like SPRI or ring resonator detection. Detecting Listeria on fish in a processing environment:
1. ****High throughput High sensitivity/low specificity monitoring**:** remote monitoring of the product, looking for micro-organism specific autofluorescence signatures. In step 1, the conveyor belt is inspected using
   (1) a dedicated light source, able to emit UV and/or blue light for generating the fluorescence,
   (2) a fluorescence-sensitive / CCD camera with filters to block the Uv/Blue excitation light and transmit the generated fluorescence light and
   (3) a processing unit to analyse images using machine vision algorithms, trained to recognize the deviating fluorescence patterns caused by the presence of microorganisms. We can continuously scan the product, looking for a suspicious surface and uses specific spectral signatures for determining whether a product must be further investigated (step 2)

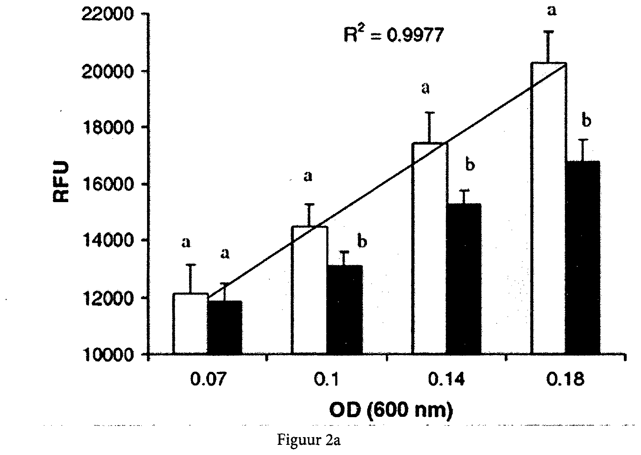

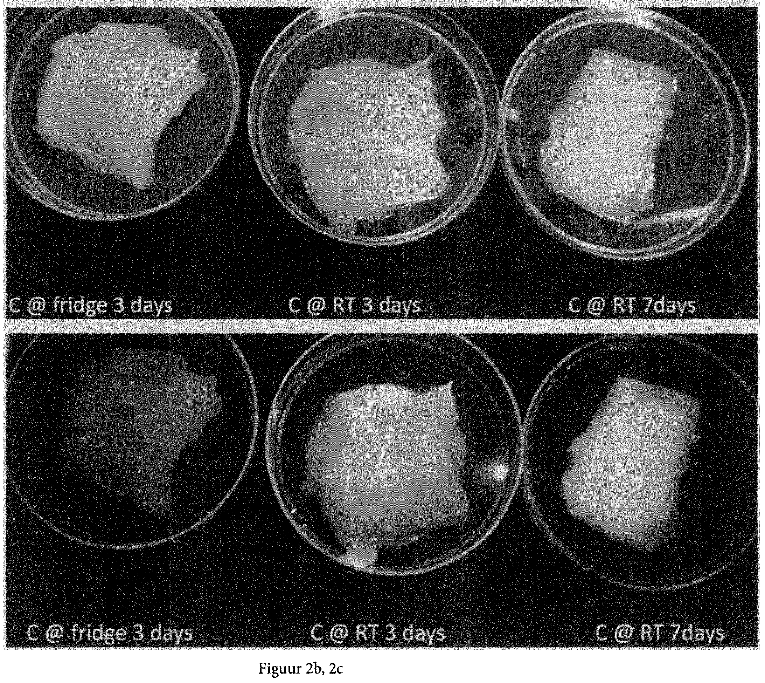

See figure 2.
2. ****Sampling**:** Use a cloth (tissue), e.g. a nitrocellulose membrane cloth, which has high affinity for biological material, sample the surfaces of the fish in the processing environment. The cloth will then contain any biological material (including the microorganisms present on the surface.
3. ****to a Liquid sample**:** The nitrocellulose cloth will be dissolved in a solution (The overarching consequence is that the nitrocellulose is soluble in organic solvents such as acetone and esters; e.g., ethyl acetate, methyl acetate, ethyl carbonate.), This process will free the biological material, including any potential Listeria bacteria, into the solvent.
4. ****Fluorescent Labeling in a container (indicative test)** **: Introduce fluorescent labels into the solution. These labels can be chosen based on their ability to bind to specific targets, such as Listeria bacteria. The container will be illuminated by a lightsource, with the specific wavelength for the chosen fluorophore and the emission will be detected using a filtered Photodetector. If fluorescence is emitted by these labels the presence of the target bacteria is likely and for more specific analysis, the next step is necessary.

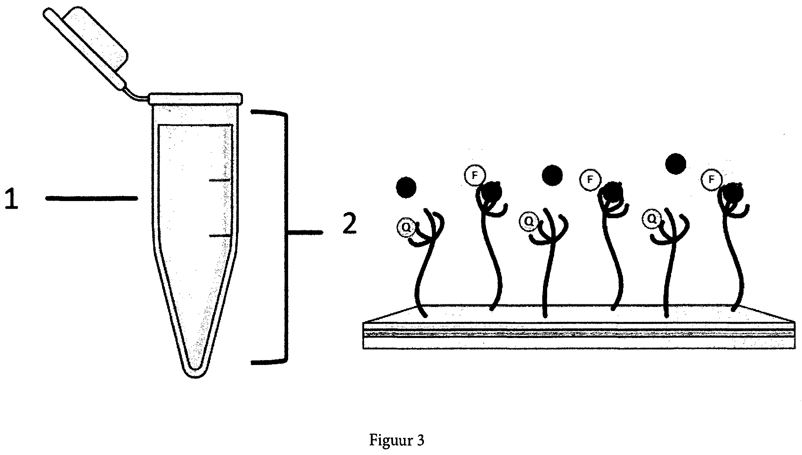

See figure 3.
https://www.mdpi.com/2079-6374/12/10/869
*i: Fluorescent component (fluorophore, e.g.see* *fig 3**)*
*ii: antibodies coupled to fluorophore ->label*
*iii: solution with sampled biological material*
*iv: reaction of target with label*
*v: fluorescence when target is present.*

*Fig 3**: from https:*//*www.mdpi.com*/*2079-6374*/*12*/*10*/*869*

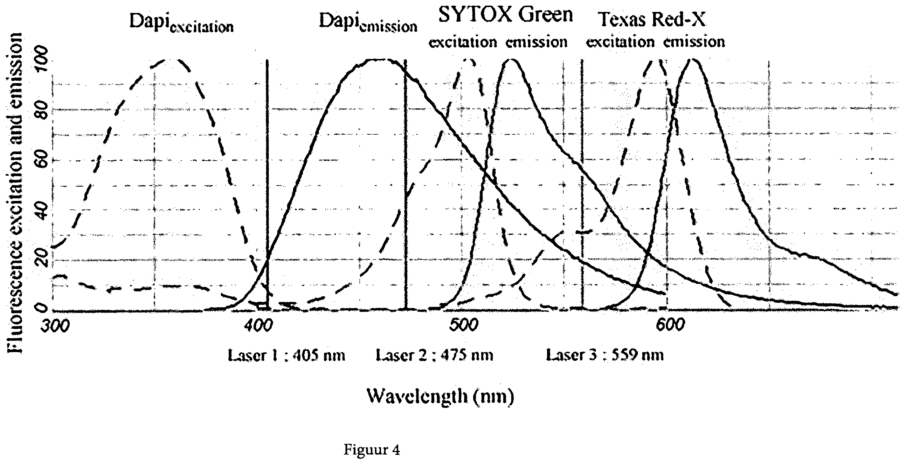

See figure 4.
5. ****Confirmatory test-high sensitivity and high selectivity** **: A surface with a number of functionalized areas, being areas of immobilized antibodies for various bacterial strains and sub-strains is used. The solvent will be running over the sensor surface and specific substances bind to their intended functionalized areas. The functionalized surface can be a gold layer (in case surface plasmon resonance imaging (SPRi), the multiplex biosensor in the top figure) is used or a photonic chip (in case a Micro Ring Resonator device (MMR) is used). Both these methods can detect changes in the functionalized surface caused by the binding of the targets, indicating the presence of (in our case) Listeria bacteria. This detection can be realized within minutes. Combinations of positive areas indicate the specific strain. The device can operate in heavy processing circumstances.
6. ****Analysis and Identification**:** Analyze the results of SPRi or MMR to determine the presence and potentially the specific strain of Listeria bacteria in the sample and thus on the fish surface. This analysis may involve analysis of the SPRi-images using machine vision algorithms (these images show which area's era positive for binding and which areas are negative, see figure below. The areas with target bound (top middle and left and lower right) show brighter in this image. Such combinations of bright images indicate a specific strain. Or analysis of binding kinetics from the MRR device.

### See: https://www.ncbi.nlm.nih.gov/pmc/articles/PMC3303711/

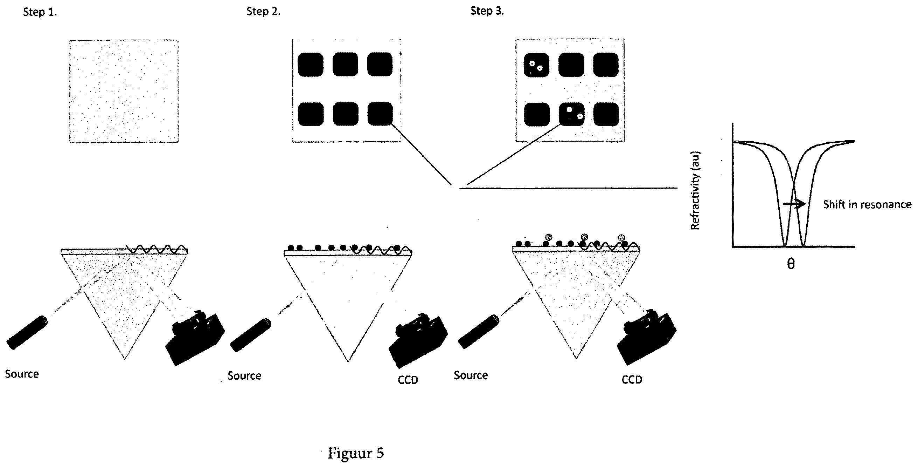

See figure 5.
6. ****Verification**:** Because of the large impact it may be necessary to verify the presence of Listeria and potentially its substrain through additional tests.
7. ****Reporting and Action**:** Report the findings and take appropriate action based on the results. This could involve implementing corrective measures in the processing environment to prevent contamination or ensuring proper handling of the fish products.

## Claims

1. A system for rapidly detecting and identifying micro-organisms on biological surfaces (e.g. fish skin surfaces) and/or non-biological surfaces (e.g. habitat or IC surfaces) comprising in combination:
a) a presumptive detection device for determining whether a surface must be further investigated, the device comprising a dedicated light source and a fluorescence-sensitive camera for scanning a surface on which microorganisms may be present i.e. a suspicious surface, the detection of microorganisms being based on the intrinsic fluorescence (auto fluorescence) properties of the micro-organisms,
b) a flexible cloth, having a high affinity for biological material and for for specific micro-organisms, and being suitable for taking a sample of the biological material including the micro-organisms on the suspicious surface e.g. by swiping over the suspicious surface,
c) a solvent for dissolving the flexible cloth together with the micro-organisms collected in or on the flexible cloth,
d) a container for containing the solvent, and for containing a solution of the solvent with a dissolved flexible cloth and dissolved micro-organisms,
e) a sensitive read out device for detecting and identifying micro-organisms, in particular also the sub- strains thereof, comprising at least one multiple area sensor, e.g. having 48 or 96 areas, whereby specific sensor means are provided on the multiple areas for binding with dissolved micro-organisms, in particular the sub-strains thereof, when the solution of the solvent with the dissolved microorganisms is running over the at least one multiple area sensor, a light source for emitting light on the at least one multiple area sensor and a detector/ identifier / CCD means able to detect and identify the dissolved micro-organisms, in particular also the sub-strains thereof, on the basis of the output of the at least one multiple area sensor.

2. System according to claim 1, **characterized in that** the system further comprises reactants which are labeled with specific fluorophores and which reactants can be activated by binding to the corresponding dissolved micro-organisms, thereby changing their fluorescence characteristic,
whereby the flexible cloth comprises the reactants and/ or the solvent comprises the reactants, and/ or the reactants are to be added to the solution in the container,
the container being further suitable for containing dissolved micro-organisms bonded to activated reactants,
the system further comprising a presumptive read out device to be used in advance of the sensitive read out device, for detecting and identifying the dissolved micro-organisms in the container, in particular main strains thereof, the device comprising a light source for exciting the fluorophores of the activated reactants and a detector/ identifier / CCD means for measuring the generated fluorescence and identifying the dissolved micro-organisms.

3. System according to claim 1 or 2, **characterized by** the flexible cloth being made of fibers, which can comprise polyester, nylon, cellulose, chitosan, peat moss, cotton, wool, hemp, corn-based fibers and/or the cloth being a membrane or (artificial) microfiber cloth or being an artificial fabric of a nitrocellulose membrane material, which has a maximal affinity for micro-organisms and is thus optimized for the adherence of microorganisms.

4. System according to any of claims 1 to 3, **characterized in that** the solvent is an organic solvent such as acetone or an ester; e.g., ethyl acetate, methyl acetate, ethyl carbonate.

5. The system according to claim 2 or any of claims 3 or 4 (when depending on claim 2), **characterized in that** the reactants are antibodies, aptamers, aptamer beacons and/ or hybridized DNA displacement assays, conjugates or phages.

6. System according to any of claims 1 to 5, **characterized in that** the container is made of a transparent or translucent material, e.g. glass or plastic.

7. System according to any of claims 1 to 6, **characterized in that** the at least one multiple area sensor is a biosensor comprising 48 or 96 functionalized areas which present different reactants, especially antibodies, for reaction with different micro-organisms.

8. System according to any of claims 1 to 7, **characterized in that** the specific sensor means comprise functionalized gold layers which are covered by small islands of reactants, especially antibodies, the detector/ identifier / CCD means making use of analyses of SPRI images (Surface Plasmon Resonance Imaging) thereby using machine vision algorithms.

9. System according to any of claims 1 to 8, **characterized in that** the specific sensor means comprise integrated light guides on which various reactants, especially antibodies, are printed, whereby a variation in light emittance is caused by binding of microorganisms to these light guides and the analysis by the detector/ identifier / CCD means is based on binding kinetics of the MMR (Mobius Micro ring Resonator).

10. System according to any of claims 1 to 9, **characterized by** the dedicated light source of the presumptive detection device is a light source able to emit UV and/or blue light and the camera of the presumptive detection device is a hyper spectral camera with e.g. a pushbroom configuration or a CCD.

11. System according to any of claims 1 to 9, **characterized by** the presumptive detection device further comprising a dedicated light source able to emit UV and/or blue light for generating the fluorescence, the fluorescence-sensitive camera comprises filters to block the UV/blue excitation light and transmits the generated fluorescence light and a processing unit to analyse images using machine vision algorithms, trained to recognize the deviating fluorescence patterns caused by the presence of microorganisms, whereby this combination is able to continuously scan a suspicious surface and uses specific spectral signatures for determining whether a surface must be further investigated.

12. The system according to any of claims 1 to 11, **characterized in that** it further comprises a conveyor belt for conveying products in a product processing facility, such as fish in a fish processing facility whereby the fish have a skin surface, or such as chicken and other meat products in a chicken and meat product processing facility, whereby the chicken and meat products have a skin surface.

13. The system according to any of claims 1 to 12, **characterized in that** the micro-organisms are bacteria such as MRSA, e- coli or listeria.

14. System comprising a flexible cloth as defined in any of claims 1-3 and 5 in combination with a solvent as defined in any of the claims 1, 2, 4 and 5 OR solution of a flexible cloth as defined in any of claims 1-3 and 5 dissolved in a solvent as defined in any of the claims 1, 2, 4 and 5, to be used as a set or part of a set for sampling.

15. Method for rapidly detecting and identifying microorganisms on biological surfaces (e.g. fish skin surfaces) and/or non-biological surfaces (e.g. habitat or IC surfaces) using the system according to any of claims 1- 15, comprising the steps of
STEP 1
1.1 scanning with a presumptive detection device a suspicious surface,
1.2 detecting and identifying microorganisms based on the intrinsic fluorescence (auto fluorescence) properties of the micro-organisms,
1.3 deciding on the basis hereof to take a sample in order to further investigate he surface,
1.4 in case of further investigation,
using a flexible cloth to take a sample of material present on the surface comprising micro-organisms, e.g. by swiping the sample means over the suspicious surface thereby adhering or absorbing micro-organisms to the flexible cloth,
1.5 providing a container with a solvent,
1.6 making a solution in the container by dissolving the flexible cloth together with the micro-organisms in the solvent,
OPTIONAL STEP 2 comprising the steps of
2.1 using at least the system according to claim 2
2.2 adding reactants labeled with specific fluorophores to the solvent or to the flexible cloth before making the solution, or adding such reactants to the solution,
2.3 using a presumptive read out device for detecting and identifying the micro-organisms, in particular the main strains thereof, and
2.4 deciding on the basis hereof to continue with further investigation of the micro-organisms,
2.5 in case of further investigation of the micro-organisms,
STEP 3
3.1 supplying the solution in the container to a sensitive read out device, and
3.2 detecting/ identifying (e.g. with a CCD) the respective specific microorganisms, in particular also the sub-strains thereof, which are present on the surface.
